# EUROPEAN PATENT APPLICATION

(11) **EP 1 894 550 A1**
(43) Date of publication of application: **05.03.2008**
(21) Application number: 07114382.0
(22) Date of filing: 15.08.2007
(51) Int. Cl.: A61F 9/01

(54) **Performance assessment system for refractive lasers and associated methods**

(30) Priority: 31.08.2006 US 513648
(71) Applicant: Alcon RefractiveHorizons, Inc., Fort Worth, Texas 76134 (US)
(72) Inventor: Liedel, Kevin K., Orlando, FL 32820 (US); Pettit, George H., Maitland, FL 32751 (US); Campin, John A., Orlando, FL 32828 (US)
(74) Representative: Hanna, Peter William Derek

(57) **Abstract**

A method for assessing a performance of an ablation laser system is provided including ablating (102) a substrate surface (13) to achieve a predetermined ablation pattern. A beam of light is directed (103) across the ablated substrate, and reflected light is received to detect an actual ablated pattern, which is compared (104) with the predetermined ablation pattern. The resulting comparison is displayed (105) to a user so that the performance of the laser system can be assessed. A system (10) includes a support (12) that is adapted to hold an ablation substrate (13) at a treatment plane. An optical scanner (19) directs a beam of light (20) across the ablated substrate (13) and receives light (21) reflected therefrom to detect an ablated pattern. An analyzer (22) compares the detected ablated pattern with the predetermined ablation pattern. A display (24) in signal communication with the analyzer displays a comparison of the detected ablated pattern and the predetermined ablation pattern.

## Description

### Field of Invention

The present invention generally relates to ophthalmic laser surgery and, in particular, to systems and methods for assessing system component performance, and, more particularly, to systems and methods for assessing the performance of the refractive laser in an ophthalmic laser surgical system.

### Background

Ophthalmic surgery for the correction of vision is known to be performed with lasers, for example, excimer lasers, which are used to ablate the cornea in a predetermined pattern. The laser is applied in bursts of energy, referred to as shots, each of which has physical and temporal characteristics that must be known precisely in order that the predetermined pattern be commensurate with the pattern ablated by the laser. Further, the optical system that directs the shots to the cornea also has physical characteristics that must be precisely calibrated in order that the shots be placed correctly.

Currently known methods for assessing laser system performance demand that the user perform a test ablation on a substrate such as plastic or paper and then remove the substrate to another device for measurement. Such assessments are typically not performed at the same position/orientation of the actual ablation.

Therefore, it would be desirable to provide a method for assessing the performance of a refractive laser system that is accurate and automated.

### Summary of the Invention

A method for assessing a performance of a laser system for use in corneal ablation is presented, wherein the method comprises the step of ablating a surface of an ablation substrate to achieve a predetermined ablation pattern. A beam of light is directed across the ablated substrate, and light reflected therefrom is received to detect an ablated pattern.

The detected ablated pattern is compared with the expected ablated pattern. A comparison of the detected ablated pattern and the expected ablated pattern is displayed to a user so that the performance of the laser system can be assessed.

A system for assessing a performance of a laser system for use in corneal ablation comprises a support that is adapted to hold an ablation substrate. An optical system directs a laser beam onto the ablation substrate at a plane of a cornea of an eye desired to be treated. An optical scanner is provided for directing a beam of light across the ablated substrate and for receiving light reflected therefrom to detect an ablated pattern.

An analyzer is adapted to compare the detected ablated pattern with the predetermined ablated pattern as it is expected to have been created. A display in signal communication with the analyzer is provided for displaying to a user a comparison of the detected ablated pattern and the expected ablation pattern.

The features that characterize the invention, both as to organization and method of operation, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

### Brief Description of the Drawing

FIG. 1 is a schematic of an embodiment of the system of the present invention.
FIG. 2 is a cross section of an expected ablation pattern.
FIG. 3 is a three-dimensional representation of a scan of an ablated material.
FIGS. 4A-4E are representations of expected (solid lines) versus actual (dotted lines for FIGS. 4A-4D) ablation patterns for different system problems: FIG. 4A illustrates a calibration problem; FIG. 4B, decentration; FIG. 4C, aberration; FIG. 4D, tilt; and FIG. 4E, rotation.
FIG. 5 is a flowchart of an exemplary method of the present invention for implementing an ablation pattern on an eye and performing a system assessment during the procedure.

### Detailed Description of the Invention

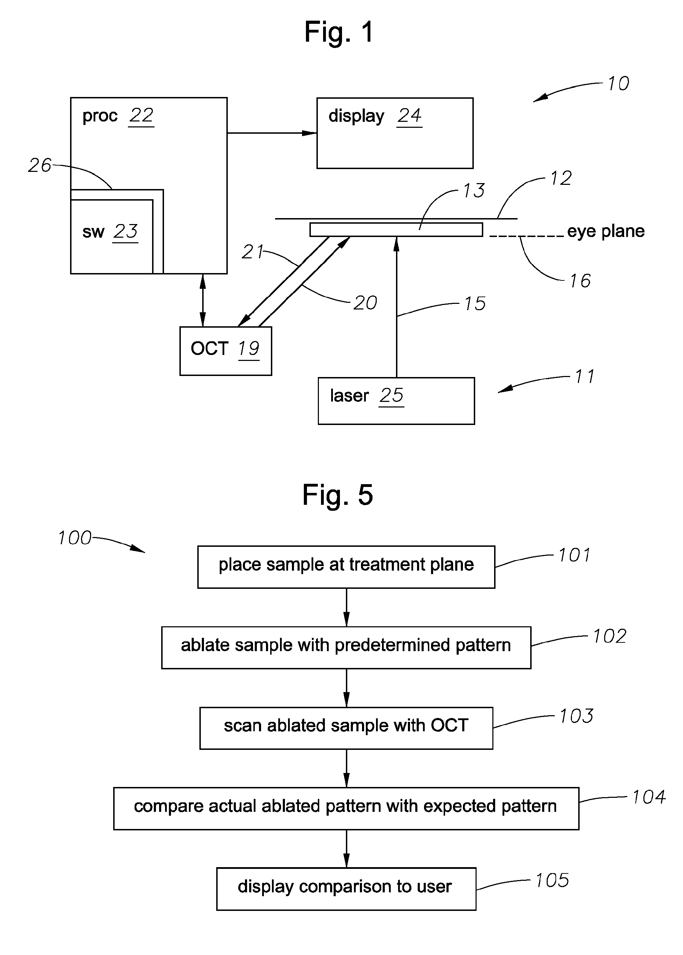

A description of the preferred embodiments of the present invention will now be presented with reference to FIGS. 1-5.

A system 10 (FIG. 1) is provided for assessing a performance of a laser system 11 for use in corneal ablation. The system 10, which is preferably completely automated, comprises a support 12 that is adapted to hold an ablation substrate 13, which may comprise, for example, a plastic material. An optical train is positioned in the path of a laser beam 15 for directing the laser beam 15 onto the ablation substrate 13 at the plane 16 of a cornea of an eye desired to be treated. This achieves the assessment's being made at the same location as the ablation itself, allowing for precise registration in x, y, and θ.

An optical coherence tomography apparatus 19 directs a beam 20 of light across the ablated substrate 13 and receives light 21 that is reflected therefrom to detect an actual ablation pattern. Reflected light 21 comprises data representative of at least the depth, curvature, and elevation of the ablated surface. A processor (analyzer) 22 with software 23 resident thereon within a memory 26 analyzes and compares the detected ablated pattern with the predetermined ablated pattern that was expected to have been created for correcting the vision of the eye.

Analyzer 22 may be a single processing device or a plurality of processing devices. Such a processing device may be a microprocessor, micro-controller, digital signal processor, microcomputer, central processing unit, field programmable gate array, programmable logic device, state machine, logic circuitry, analog circuitry, digital circuitry, and/or any device that manipulates signals (analog and/or digital) based on operational instructions. The memory 26 may be a single memory device or a plurality of memory devices. Such a memory device may be a read-only memory, random access memory, volatile memory, non-volatile memory, static memory, dynamic memory, flash memory, cache memory, and/or any device that stores digital information. Note that when the analyzer 22 implements one or more of its functions via a state machine, analog circuitry, digital circuitry, and/or logic circuitry, the memory 26 storing the corresponding operational instructions (software 23) may be embedded within, or external to, the circuitry comprising the state machine, analog circuitry, digital circuitry, and/or logic circuitry. The memory 26 stores, and the analyzer 22 executes, operational instructions (e.g., software 23) corresponding to at least some of the steps and/or functions illustrated in the FIGS.

A display 24 in signal communication with the analyzer 22 displays to a user a comparison of the detected ablated pattern and the expected ablated pattern. Among the possible displays are a profile of the ablated profile, a difference plot between the detected and the expected ablation pattern, a cross section of the ablated profile, and a three-dimensional view of the ablated profile, although these are not intended to be limiting. For example, an easily interpreted three-dimensional confirmation of ablation accuracy can be provided for the user

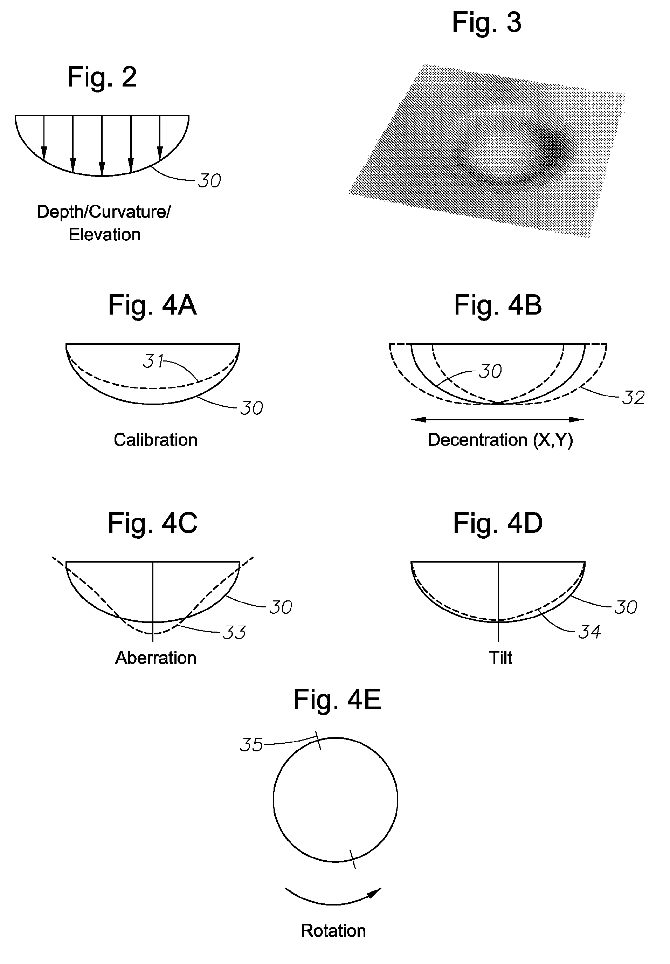

As an example, take an expected ablation profile 30 that comprises a crater having a depth, curvature, and elevation, such as shown in FIG. 2 in cross section, which represents a simple spherical profile, shown in FIG. 3 in a three-dimensional view. Several types of departures may occur, as shown in FIGS. 4A-4E. In FIG. 4A, the actual ablated profile 31 (dashed line) is shown to be shallower than the expected profile 30, likely caused by a calibration error. In FIG. 4B, the actual ablated profile 32 is shown to have a centration error in the x,y direction. In FIG. 4C, the shape of the actual ablated profile 33 is incorrect, caused by an aberration. In FIG. 4D, the actual ablated profile 34 is asymmetric, caused by tilt. In FIG. 4E, there is a rotation error in the actual ablated profile 35.

A method 100 for assessing a performance of a laser system for use in corneal ablation (FIG. 5) includes placing at the treatment plane a flat plastic sample 13 having a suitable surface quality and ablation characteristics (block 101). The laser 25 is used to ablate the surface 26 of the ablation sample 13 to achieve a predetermined ablation pattern (block 102).

An optical coherence tomography apparatus 19 is used to scan the ablated sample 13 (block 103) and to compare the detected (actual) ablated pattern with the expected ablated pattern (block 104). Any or all of the possible display types outlined above can then be displayed to the user (block 105) to indicate a comparison of the detected ablated pattern and the expected ablated pattern. Thus an assessment of the intended ablation can be made in substantially the exact position/orientation of the actual ablation, allowing for a more detailed calibration.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiments thereof, and the advantageous new and useful results obtained thereby, the new and useful constructions, and reasonable mechanical equivalents thereof obvious to those skilled in the art, are set forth in the appended claims.

## Claims

1. A system (10) for assessing a performance of a laser system (25) for use in corneal ablation, the system comprising:
a support (12) adapted to hold an ablation substrate (13);
an optical system for directing a laser beam onto the ablation substrate in a plane (16) of a cornea of an eye desired to be treated to ablate the ablation substrate in a predetermined pattern;
an optical scanner (19) for directing a beam of light (20) across the ablated substrate (13) and for receiving light (21) reflected therefrom to detect an actual ablated pattern;
an analyzer (22) for comparing the detected ablated pattern with the predetermined ablation pattern; and
a display (24) in signal communication with the analyzer for displaying to a user a comparison of the detected ablated pattern and the predetermined ablation pattern.

2. The system recited in Claim 1, wherein the ablation substrate (13) comprises a plastic material.

3. The system recited in Claim 1, wherein the optical scanner comprises an optical coherence tomography apparatus (19).

4. The system recited in Claim 1, wherein the analyzer (22) and the display (24) are adapted to display at least one of a profile of the ablated pattern, a difference plot between the detected and the predetermined ablation pattern, and a cross section of the ablated profile, and a three-dimensional view of the ablated profile.

5. A method for assessing a performance of a laser system (25) or use in corneal ablation, the method comprising the steps of:
ablating (101) a surface of an ablation substrate (13) to achieve a predetermined ablation pattern;
directing a beam of light (103) across the ablated substrate and receiving light reflected therefrom to detect an actual ablated pattern;
comparing (104) the detected ablated pattern with the predetermined ablation pattern; and
displaying (105) to a user a comparison of the detected ablated pattern and the predetermined ablation pattern.

6. The method recited in Claim 5, wherein the ablation substrate (13) comprises a plastic material.

7. The method recited in Claim 5, wherein the directing step (103) comprises using an optical coherence tomography apparatus (19).

8. The method recited in Claim 5, wherein the displaying step (105) comprises displaying at least one of a profile of the ablated pattern, a difference plot between the detected and the predetermined ablation pattern, and a cross section of the ablated profile, and a three-dimensional view of the ablated profile.
